Europäisches Patentamt

European Patent Office

Office européen des brevets

(11) Veröffentlichungsnummer: **0 453 741 A2**

# (12) EUROPÄISCHE PATENTANMELDUNG

(21) Anmeldenummer: **91103496.5**

(22) Anmeldetag: **07.03.91**

(51) Int. Cl.⁵: **G01N 29/00**

(30) Priorität: **07.03.90 DE 4007206**

(43) Veröffentlichungstag der Anmeldung:
**30.10.91 Patentblatt 91/44**

(84) Benannte Vertragsstaaten:
**CH DE FR GB IT LI NL**

(71) Anmelder: **Kaltschmidt, Horst, Prof.Dr.**
**Nibelungenstrasse 2**
**W-8014 Neubiberg(DE)**

Anmelder: **Kaltschmidt, Christian**
**Keplerstrasse 9**
**W-8033 Martinsried(DE)**

Anmelder: **Kaltschmidt, Barbara**

**Keplerstrasse 9**
**W-8033 Martinsried(DE)**

(72) Erfinder: **Kaltschmidt, Horst, Prof.Dr.**
**Nibelungenstrasse 2**
**W-8014 Neubiberg(DE)**
Erfinder: **Kaltschmidt, Christian**
**Keplerstrasse 9**
**W-8033 Martinsried(DE)**
Erfinder: **Kaltschmidt, Barbara**
**Keplerstrasse 9**
**W-8033 Martinsried(DE)**

(74) Vertreter: **Schwan, Gerhard, Dipl.-Ing.**
**Elfenstrasse 32**
**W-8000 München 83(DE)**

(54) **Verfahren und Vorrichtung zum Ermitteln eines für eine Basenpaarsequenz spezifischen Schwingungsgemisches und/oder zum Beaufschlagen von DNA- oder RNA-Molekülen mit einem solchen Schwingungsgemisch.**

(57) Verfahren und Vorrichtung zum Ermitteln eines für eine Basenpaarsequenz spezifischen Schwingungsgemisches und/oder zum Beaufschlagen von DNA- oder RNA-Molekülen mit einem solchen Schwingungsgemisch, bei denen DNA- bzw. RNA-Material Schwingungen unterschiedlicher Frequenz ausgesetzt oder zu Eigenschwingungen angeregt wird und die zu Resonanzschwingungen der Moleküle führenden spezifischen Schwingungsgemische für bekannte Basenpaarsequerzen bestimmt werden, oder bei denen DNA- oder RNA-Moleküle auf das Vorhandensein von bestimmten Basenpaarsequenzen getestet werden, indem festgestellt wird, ob bestimmte basenpaarsequerzspezifische Schwingungsgemische Resonarzschwingungen auslösen, oder bei denen auf DNA- oder RNA-Moleküle an vorgewählter Strangstelle dadurch eingewirkt wird, daß die Moleküle mit einem basenpaarsequenz-spezifischen Schwingungsgemisch zu Resonanzschwingungen angeregt werden.

FIG. 10

Die Erfindung betrifft ein Verfahren und eine Vorrichtung zum Ermitteln eines für eine Basenpaarsequenz von DNA- oder RNA-Molekülen spezifischen Schwingungsgemisches und/oder zum Beaufschlagen von DNA- oder RNA-Molekülen mit einem solchen Schwingungsgemisch.

Ein DNA-Molekül weist bekanntlich zwei Einzelstränge aus sich regelmäßig wiederholenden Grundbausteinen eines Pentose-Zucker-Moleküls und einer Phosphatgruppe auf. Die Einzelstränge sind in Form einer Doppelspirale (Doppel-Helix) ineinander gewunden. Die Basenpaare Adenin-Thymin (A-T oder T-A) und Guanin-Cytosin (G-C oder C-G) befinden sich - vergleichbar mit den Sprossen einer Leiter - zwischen den beiden Einzelsträngen (Leiterholmen). Ein RNA-Molekül unterscheidet sich von einem DNA-Molekül prinzipiell nur dadurch, daß Uracil (U) an die Stelle des Thymins (T) tritt und daß der Zuckerring im Rückgrat des RNA-Moleküls nicht aus Desoxyribose, sondern aus Ribose besteht.

Es ist bekannt (u.a. Römpps Chemie-Lexikon, 8. Auflage, 1987, Band 5, Seiten 3812 und 3813 mit weiteren Literaturverweisen), zur Bestimmung der Reihenfolge der Basenpaare eines DNA-Moleküls Desoxiribonukleinsäuren zunächst mittels Restriktionsendonukleasen in überlappende Bruchstücke zu zerlegen. Dabei wird nach der von Maxam und Gilbert entwickelten Methode ein radioaktiv markierter DNA-Strang chemisch gespalten, indem auf ihn für die vier Basen Adenin (A), Guanin (G), Cytosin (C) und Thymin (T) jeweils spezifische Reagenzien einwirken läßt. Entsprechend der Methode von Sanger wird dagegen ein der zu untersuchenden Einzelstrang-DNA komplementärer Strang aufgebaut, wobei jeweils eines der vier benötigten Nukleosidtriphosphate radioaktiv markiert ist. Bei beiden Methoden entstehen verschieden lange DNA-Stücke, die durch Polyacrylamid-Gel-Elektrophorese getrennt und mittels Autoradiographie sichtbar gemacht werden. Angesichts der oft enorm großen Anzahl von Nukleotiden (z.B. 3 Milliarden bei dem bisher nur in Teilen entschlüsselten menschlichen Genom) sind solche bekannte Sequenzanalysen sehr aufwendig und zeitraubend.

Es ist auch bekannt, daß DNA- und RNA-Doppelspiralen lokal zum Schmelzen gebracht werden können, indem ihnen Schwingungsenergie zugeführt wird (Physics Letters, Vol. 70A, No. 5,6,2. April 1979, Seiten 492 bis 494; J. Chem. Phys., Band 80, No. 12,15. Juni 1984, Seiten 6291 bis 6298; Physical Review Letters, Band 62, No. 23,5. Juni 1989, Seiten 2755 bis 2758).

Der Erfindung liegt die Aufgabe zugrunde, ein Verfahren und eine Vorrichtung zu schaffen, die eine relativ einfache Sequenzanalyse von DNA- und RNA-Molekülen erlauben. Es sollen ferner ein Verfahren und eine Vorrichtung geschaffen werden, mittels deren DNA- und RNA-Moleküle anhand der mittels Sequenzanalyse gewonnenen Ergebnisse gezielt beeinflußt werden können.

Gegenstand der Erfindung ist entsprechend einem ersten Aspekt ein Verfahren zum Ermitteln eines für eine bestimmte Basenpaarsequenz von DNA- oder RNA-Molekülen spezifischen Schwingungsgemisches nach Amplitude und Phase, bei dem das DNA- bzw. RNA-Material, das die bestimmte Basenpaarsequenz hat, Schwingungen unterschiedlicher Frequenz ausgesetzt oder zu Eigenschwingungen angeregt wird und die Frequenz oder Frequenzen nach Amplitude und Phase ermittelt werden, bei denen in dem DNA- bzw. RNA-Material Resonanzschwingungen auftreten. Mittels einer solchen Frequenzmusteranalyse lassen sich Daten gewinnen, anhand deren DNA- oder RNA-Moleküle dann auf das Vorhandensein von bestimmten Basenpaarsequenzen untersucht werden können und/oder die es erlauben, an vorgewählter Strangstelle auf DNA- oder RNA-Moleküle einzuwirken.

Gemäß einem weiteren Aspekt wird erfindungsgemäß ein Verfahren zum Untersuchen von DNA- oder RNA-Molekülen auf das Vorhandensein einer bestimmten Basenpaarsequenz geschaffen, bei dem die Moleküle durch erzwungene Schwingungen oder Eigenschwingungen mit einem nach Amplitude und Phase vorbestimmten Schwingungsgemisch angeregt werden, das spezifisch für die gesuchte Basenpaarsequenz ist, und bei dem ermittelt wird, ob beim Anregen mit diesem Schwingungsgemisch Resonanzschwingungen auftreten. Dieses Vorgehen erlaubt es festzustellen, ob in einem DNA- oder RNA-Molekül oder in einem Bruchstück eines solchen Moleküls eine vorgegebene Basenpaarsequenz vorliegt oder nicht.

Mit der Erfindung wird ferner ein Verfahren geschaffen, bei dem auf DNA- oder RNA-Moleküle dadurch an vorgewählter Strangstelle eingewirkt wird, daß die Moleküle mit einem nach Amplitude und Phase vorbestimmten, basenpaarsequenz-spezifischen Gemisch von erzwungenen Schwingungen oder Eigenschwingungen beaufschlagt werden. Dabei kann insbesondere den DNA- oder RNA-Molekülen mittels des basenpaarsequenz-spezifischen Schwingungsgemisches eine Energiemenge zugeführt werden, die einen Strangbruch an der vorgewählten Strangstelle verursacht.

Entsprechend einer abgewandelten Ausführungsform der Erfindung werden die DNA- oder RNA-Moleküle an einem ersten Sequenzabschnitt, der eine bekannte Sequenz von n Basenpaaren hat, durch ein für diese basenpaarsequenz-spezifisches Schwingungsgemisch mit einer nicht zum Strangbruch führenden Energie zu Resonanzschwingungen angeregt; das Spektrum der in den DNA- bzw. RNA-Molekülen angeregten Resonanzschwingungen wird nach Amplitude und Phase ermittelt und mit bekannten Resonanzschwingungsspektren(Mustern) verglichen, die spezifisch für eine Sequenz von $n + m$ Basenpaaren sind, wobei $n$ und $m$ ganze Zahlen sind; anhand dieses Vergleichs wird die Basenpaarsequenz für einen zweiten Sequenzabschnitt ermittelt, der gegenüber dem ersten Sequenzabschnitt entlang dem Strang um $m$ Basenpaare versetzt ist; die DNA- bzw. RNA-Moleküle werden dann mit einem nicht zum Strangbruch führenden Schwingungsgemisch angeregt, das spezifisch für die Basenpaarsequenz des zweiten Sequenzabschnittes ist; die vorgenannten Verfahrensschritte werden bedarfsweise wiederholt. Dieses Vorgehen gestattet eine relativ einfache Sequenzanalyse auf physikalischem Wege, die sich auch für eine teil- oder vollautomatisierte Durchführung eignet. Besonders zweckmäßig ist es, das DNA- oder RNA-Material während der Behandlung oder Untersuchung abzukühlen oder im hydrolisierten Zustand zu behandeln und/oder zu untersuchen, um auf diese Weise störende Wärmeschwingungen klein zu halten. Zusätzlich oder stattdessen, beispielsweise im Falle einer Behandlung des DNA- oder RNA-Materials in wäßriger lösung, können vorteilhaft die DNA- oder RNA-Moleküle mittels eines inhomogenen elektrischen Gleichfeldes weitgehend parallel zueinander ausgerichtet werden. Dies führt zu einer Verstärkung der Nutzschwingungen durch Gleichschaltung einer größeren Anzahl von Molekülen.

Die Beaufschlagung des DNA- oder RNA-Materials mit Schwingungen und/oder die Messung des basensequenz-spezifischen Resonanzschwingungsspektrums können mechanisch, insbesondere vibratorisch oder akustisch, erfolgen. Entsprechend abgewandelten Ausführungsformen des erfindungsgemäßen Verfahrens und in Abhängigkeit von den Frequenzen der basenpaarsequenz-spezifischen Resonanzschwingungen kann das DNA- oder RNA-Material zu erzwungenen Schwingungen oder zu Eigenschwingungen durch kapazitive, magnetische, elektromagnetische oder Strahlungsankopplung angeregt werden.

Eine zur Durchführung der Verfahrensvarianten nach den Ansprüchen 1 und 2 bestimmte Vorrichtung weist erfindungsgemäß eine DNA- oder RNA-Moleküle zu erzwungenen Schwingungen oder Eigenschwingungen mit variabler Frequenz veranlassende Anregungseinrichtung sowie eine Meßeinrichtung zum Ermitteln des Schwingungsgemisches nach Amplitude und Phase auf, bei dem die DNA- bzw. RNA-Moleküle in Resonanzschwingungen kommen. Dabei kann das Anregen zu erzwungenen Schwingungen mit unterschiedlichen Frequenzen grundsätzlich zeitlich sequentiell (seriell) oder zeitgleich (parallel) erfolgen.

Eine Vorrichtung zur Durchführung des Verfahrens nach Anspruch 3 ist erfindungsgemäß gekennzeichnet durch eine Anregungseinrichtung, die DNA- oder RNA-Moleküle zu Resonanzschwingungen veranlaßt.

Die Anregungseinrichtung kann in weiterer Ausgestaltung der Erfindung einen Mustergenerator zur Herbeiführung von erzwungenen Schwingungen der DNA- oder RNA-Moleküle mit einem nach Amplitude und Phase vorbestimmten oder einstellbaren Schwingungsspektrum aufweisen. Entsprechend einer abgewandelten Ausführungsform der Erfindung ist die Anregungseinrichtung als Anordnung zum Entdämpfen des von den DNA- oder RNA-Molekülen gebildeten schwingungsfähigen Systems ausgelegt.

Bevorzugte Ausführungsbeispiele der Erfindung sind nachstehend anhand der beiliegenden Zeichnungen näher erläutert. Es zeigen:

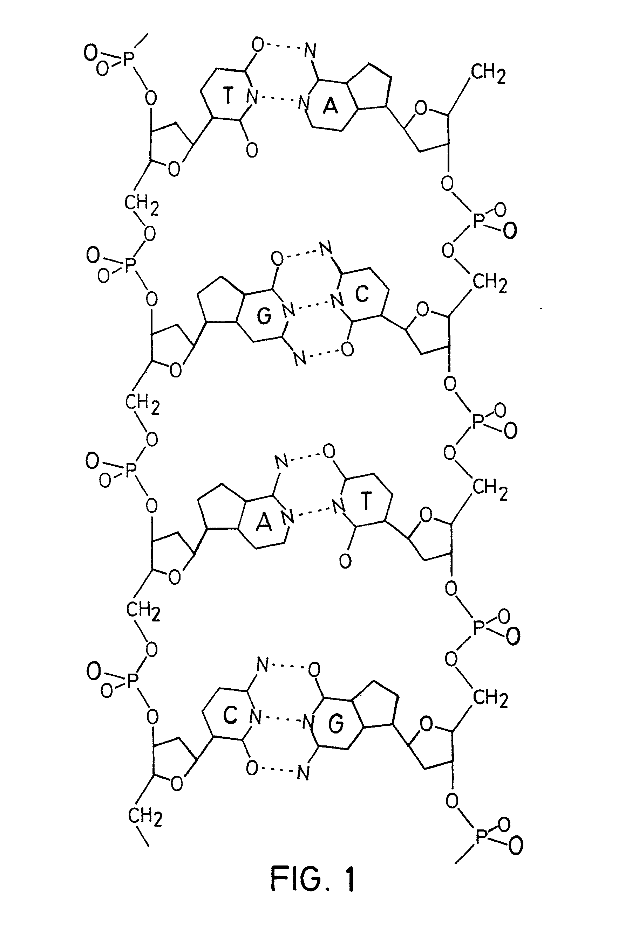

Fig. 1
ein Stück eines DNA-Moleküls,
Fig. 2
ein stark vereinfachtes ebenes Schwingungsersatzbild für das DNA-Stück gemäß Fig. 1,
Fig. 3
ein stark vereinfachtes Vertikal-Schwingungsersatzbild für das DNA-Teilstück der Fig. 1,
Fig. 4
eine schematische Darstellung einer elektroakustischen DNA-Resonanzmeßvorrichtung,
Fig. 5
eine schematische Meßanordnung für den radarfrequenten Bereich mit Mitteln zum Ausrichten der DNA-Moleküle,
Fig. 6
ein schematisches Schaltbild eines rückgekoppelten Verstärkersystems zur Ausbildung elektrischer angepaßter Signale,
Fig. 7
eine magnetische Koppelanordnung zur Kopplung eines DNA-Volumens mit einer elektromagnetischen Sende/Empfangs-Einrichtung im Millimeter- und Submillimeter-Bereich,
Fig. 8
eine schematische Darstellung einer Vorrichtung zur Reflex-Strahlungskopplung,
Fig. 9
eine schematische Anordnung einer als Hohlleitersystem ausgebildeten Multiresonator-Koppelanordnung,
Fig. 10
eine schematische Darstellung einer in Anlehnung an Laser-/Maser-Prinzipien aufgebauten Vorrichtung zur Erzeugung und Messung von sequenzangepaßten Signalen,
Fign. 11 und 12
schematische Darstellungen von zwei mit Elektronenstrahlkopplung arbeitenden Vorrichtungen, und

Fig. 13

ein Beispiel einer DNA-Basensequenz.

Fig. 1 zeigt ein Stück eines DNA-Moleküls. Die in dieser Figur rechts und links dargestellten Zucker-Phosphat-Holme laufen in entgegengesetzten Richtungen. Jede Basenpaar-Sprosse besteht bekanntlich aus einem Purin, nämlich Adenin oder Guanin, und einem Pyrimidin, nämlich Thymin oder Cytosin, die über Wasserstoffbrücken (als Punkte dargestellt) verknüpft sind.

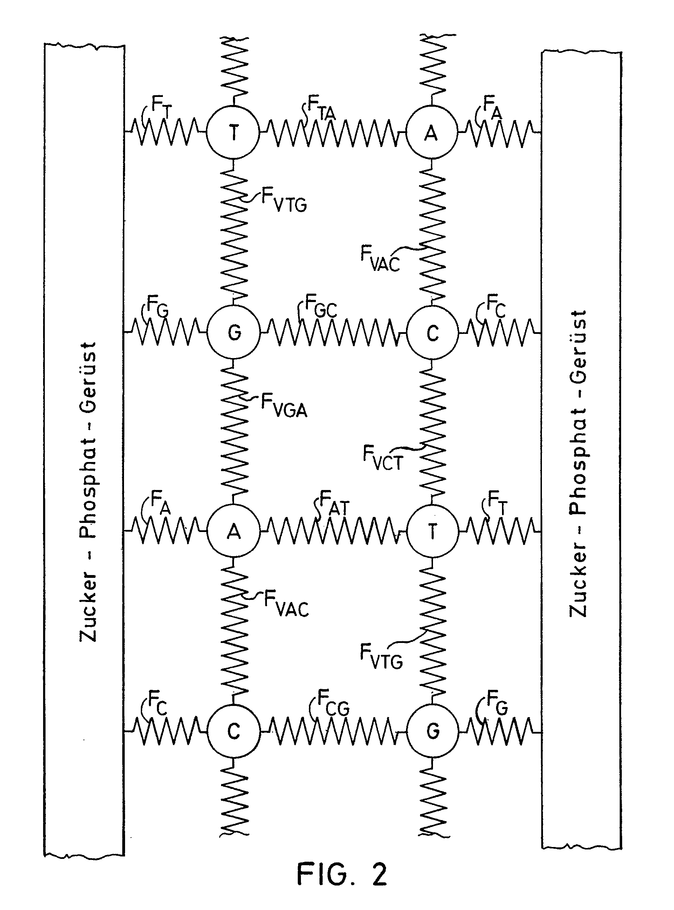

Die vorstehend erläuterten Vorgehensweisen beruhen auf der Erkenntnis, daß DNA- und RNA-Moleküle sowie deren Bruchstücke ein komplexes, gekoppeltes Schwingungssystem bilden. Ein stark vereinfachtes, ebenes, mechanisches Schwingungsersatzbild des DNA-Molekülstücks gemäß Fig. 1 ist in Fig. 2 wiedergegeben. Dabei ist davon ausgegangen, daß die Moleküle der Basen Adenin (A), Guanin (G), Cytosin (C) und Thymin (T) der einzelnen Nukleotide als konzentrierte Massen $m_A$, $m_G$, $m_C$ bzw. $m_T$ entsprechend den betreffenden Molekülgewichten dargestellt werden können, sowie daß sich die Bindungen zwischen den einzelnen Basen und dem Zucker-Phosphat-Gerüst sowie zwischen den Basen jedes Basenpaares als Federn betrachten lassen. Die Federn, über welche die konzentrierten Massen der Moleküle A, G, C und T an dem zunächst als steif angenommenen Zucker-Phosphat-Gerüst hängen, sind in Fig. 2 mit $F_A$, $F_C$, $F_G$ bzw. $F_T$ bezeichnet; sie haben die Federkonstanten $D_A$, $D_C$, $D_G$ bzw. $D_T$. $F_{TA}$, $F_{GC}$, $F_{AT}$ und $F_{CG}$ sind in Fig. 2 die Federn, die innerhalb jedes Basenpaares die einzelnen Basen mit der jeweils komplementären Base verbinden und die die Federkonstanten $D_{TA} = D_{AT}$ und $D_{GC} = D_{CG}$ haben. Wegen der kleinen gegenseitigen Abstände der Basenpaare entlang dem Strang sind auch Kräfte zwischen den in Strangrichtung aufeinanderfolgenden Basenpaaren zu erwarten, die als weitere Federn $F_{VTG}$, $F_{VAC}$, $F_{VGA}$ und $F_{VCT}$ mit den Federkonstanten $D_{VTG}$, $D_{VAC}$, $D_{VGA}$ bzw. $D_{VCT}$ darstellbar sind. Insgesamt gibt es bei einem DNA-Molekül grundsätzlich vier Basenpaarungen, und zwar T-A, A-T, G-C und C-G, wobei jede Basenpaarung mit vier verschiedenen Nachbarn (in Strangrichtung) auftreten kann, denen ebenfalls verschiedene Federkonstanten zuzuordnen sind. Nachdem schon ein einfaches räumliches Feder-Masse-System mit einer einzigen Masse und einer einzigen Feder über drei translatorische und drei rotatorische Freiheitsgrade verfügt, versteht es sich, daß selbst ein relativ kurzes DNA-Molekül ein System mit einer Vielzahl von Freiheitsgraden bildet.

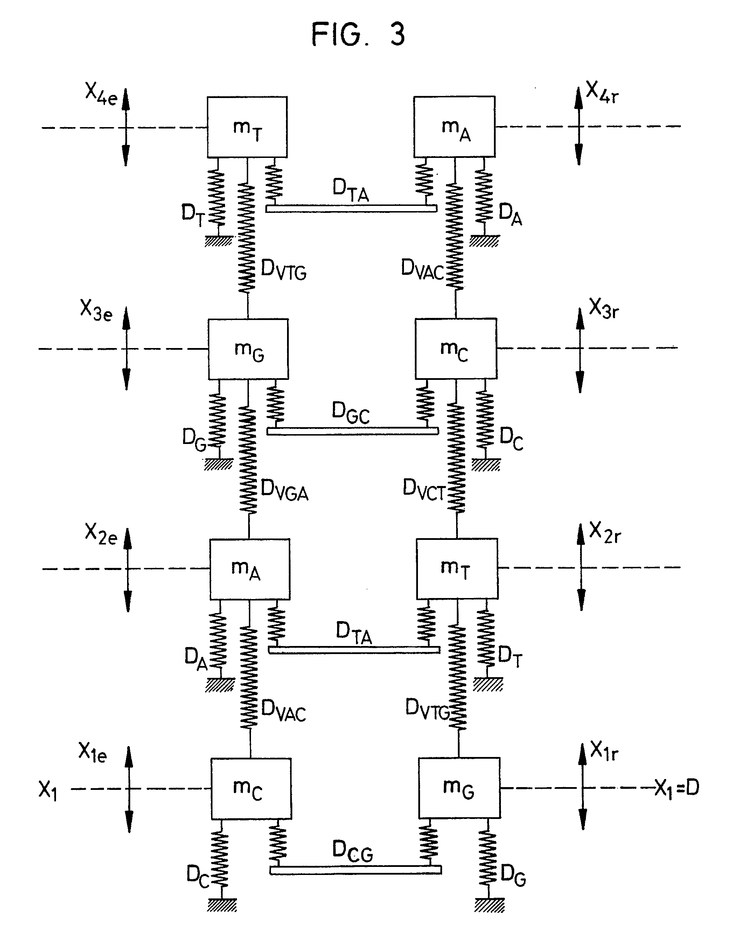

Ausgehend von der Schwingungssystemanalogie der Fig. 2 läßt sich jedoch das stark vereinfachte Schwingungsersatzbild gemäß Fig. 3 für ebene Vertikalschwingungen des DNA-Abschnittes der Fig. 1 unter der grob zu treffenden Annahme aufstellen, daß das Zucker-Phosphat-Gerüst starr ist und daß seine Masse gegenüber der Masse der einzelnen Basen so groß ist, daß sich das Gerüst unter dem Einfluß von Schwingungen nicht oder fast nicht bewegt. Dabei ist in Fig. 3 die Gleichgewichtslage der einzelnen Basen gegenüber dem Zucker-Phosphat-Gerüst jeweils durch eine gestrichelte Linie angedeutet. Vertikalverschiebungen der konzentrierten Massen $m_T$, $m_G$, $m_A$ und $m_C$ gegenüber der Gleichgewichtslage sind mit $X_{1e}$, $X_{2e}$, $X_{3e}$, $X_{4e}$ bzw. $X_{1r}$, $X_{2r}$, $X_{3r}$ und $X_{4r}$ bezeichnet. Für den Energieverzehr verantwortliche Dämpfungsglieder sind weggelassen. Weiterhin ist Linearität vorausgesetzt, die nur für Kleine Schwingungsamplituden zu erwarten ist.

Aus Fig. 3 erhält man das folgende Schwingungsgleichungssystem:

$$m_G \cdot X_{1r} + D_G X_{1r} + D_{VTG}(X_{1r} - X_{2r}) + D_{GC}(X_{1r} - X_{1e}) = 0 \qquad (1)$$

$$m_C \cdot X_{1e} + D_C X_{1e} + D_{VAC}(X_{1e} - X_{2e}) + D_{GC}(X_{1e} - X_{1r}) = 0 \qquad (2)$$

$$m_T \cdot X_{2r} + D_T X_{2r} + D_{VTG}(X_{2r} - X_{1r}) + D_{VCT}(X_{2r} - X_{3r}) + D_{TA}(X_{2r} - X_{2e}) = 0 \qquad (3)$$

$$m_T \cdot X_{4e} + D_T X_{4e} + D_{VTG}(X_{4e} - X_{3e}) + D_{TA}(X_{4e} - X_{4r}) = 0 \qquad (4)$$

Das gekoppelte Schwingungssystem gemäß Fig. 3 hat acht Resonanzfrequenzen, deren Werte von den einzelen Massen und ihren Kopplungen abhängen. Mithin ist das Resonanzfrequenz-Muster sequenzspezifisch. Erregt man das System mit einem Schwingungsgemisch, dessen Einzelschwingungsfrequenzen dem Resonanzfrequenzmuster entsprechen, oder veranlaßt man das System zu entsprechenden Eigenschwingungen, nimmt das System maximale Energie auf, während benachbarte Schwingungssysteme verhältnismäßig wenig Energie aufnehmen. Die zugehörige Schwingungsform kann man - in Anlehnung an die Begriffe der Nachrichtentechnik - "angepaßtes Signal" nennen. Insofern ist es zum Beispiel möglich, eine bestimmte Stelle des DNA-Moleküls, wie die TGAC-Sequenz der Fig. 1, zum "Kochen" zu bringen, während andere Stellen des DNA-Moleküls kühl bleiben. Durch genügend hohe, an die Basensequenz angepaßte Energiezufuhr mittels angepaßter Signale ist es möglich, in einem Gemisch von DNA-Molekülen, die sich durch einen speziellen Sequenzabschnitt unterscheiden, nur jene zu zerstören, bei denen innerhalb dieses Sequenzabschnittes eine vorbestimmten Basensequenz vorliegt. Bei Herbeiführung eines Strangbruchs muß man mit so großen Amplituden der Basenpaare um die Ruhelage rechnen, daß das Schwingungssystem nichtlinear wird. Das bedeutet, daß zusätzliche Harmonische auftreten werden, bzw. daß auch auf den entsprechenden Spektrallinien dieser Harmonischen zusätzlich Energie zugeführt werden muß, um den Strangbruch unter dem Einfluß von Resonanzschwingungen zu erreichen. Wegen der Komplexität des Schwingungssystems, das in Wirklichkeit ein multigekoppeltes, multifreiheitsgradiges Schwingungssystem darstellt, wird die theoretische Schwingungsanalyse, jedenfalls nach dem derzeitigen Kenntnisstand nur Hinweise darauf geben können, welches (zeit- und amplitudenabhängige) Signalgemisch (Frequenzen, Amplituden, Phasen) auf DNA- bzw. RNA-Moleküle einwirken muß, um diese sequenzspezifisch zu Resonanzschwingungen zu veranlassen. Vielmehr erscheint derzeit die Ermittlung sequenzangepäßter Signale nur meßtechnisch möglich.

Die hier vorgeschlagene physikalisch-nachrichtentechnische Methode geht davon aus, daß das gemessene Schwingungsgemisch (ein komplexes Spektrum mit den Parametern: Amplitude, Frequenz und Phase) als Muster aufgefaßt werden kann, das mit einer Abfolge von Basenpaaren eindeutig korrespondiert. Um ein solches für eine bestimmte Basenpaarsequenz spezifisches Schwingungsgemisch (das "angepaßte Signal") zu ermitteln, wird ein DNA- oder RNA-Material, das eine vorbekannte, durch konventionelle Sequenzanalyse bestimmte Basensequenz hat, zeitlich sequentiell (seriell) oder zeitgleich (parallel) Schwingungen unterschiedlicher Frequenz ausgesetzt oder zu Eigenschwingungen angeregt. Dabei werden die Frequenz oder Frequenzen nach Amplitude und Phase erfaßt, bei denen in dem DNA- bzw. RNA-Material Resonanzschwingungen auftreten. Derartige Resonanzschwingungen sind insbesondere an einer maximalen Energieaufnahme durch das Versuchsmaterial mit der bekannten Basenpaarsequenz zu erkennen. Das angepaßte Signal kann von der Anregungsintensität abhängig sein. Insbesondere sind bei geringer Anregungsstärke - und dementsprechend im linearen Bereich erfolgenden Molekülschwingungen - gleiche Ergebnisse für serielle wie für parallele Anregung zu erwarten. Ist die Anregungsintensität dagegen so hoch, daß Schwingungen im nichtlinearen Bereich erfolgen, werden sich Unterschiede zwischen zeitsequentieller und zeitgleicher Anregung mit verschiedenen Frequenzen einstellen.

Nachdem auf diese oder andere Weise die angepaßten Signale für unterschiedliche Basenpaarsequenzen gefunden sind, können noch nicht sequenzanalysierte DNA- oder RNA-Moleküle darauf untersucht werden, ob eine bestimmte Basenpaarsequenz vorliegt, indem die Moleküle durch erzwungene Schwingungen oder durch Eigenschwingungen mit dem für diese Sequenz spezifischen Schwingungsgemisch angeregt werden. Zeigen sich Resonanzschwingungen, ist dies ein Anzeichen dafür, daß die gesuchte Basenpaarsequenz vorhanden ist.

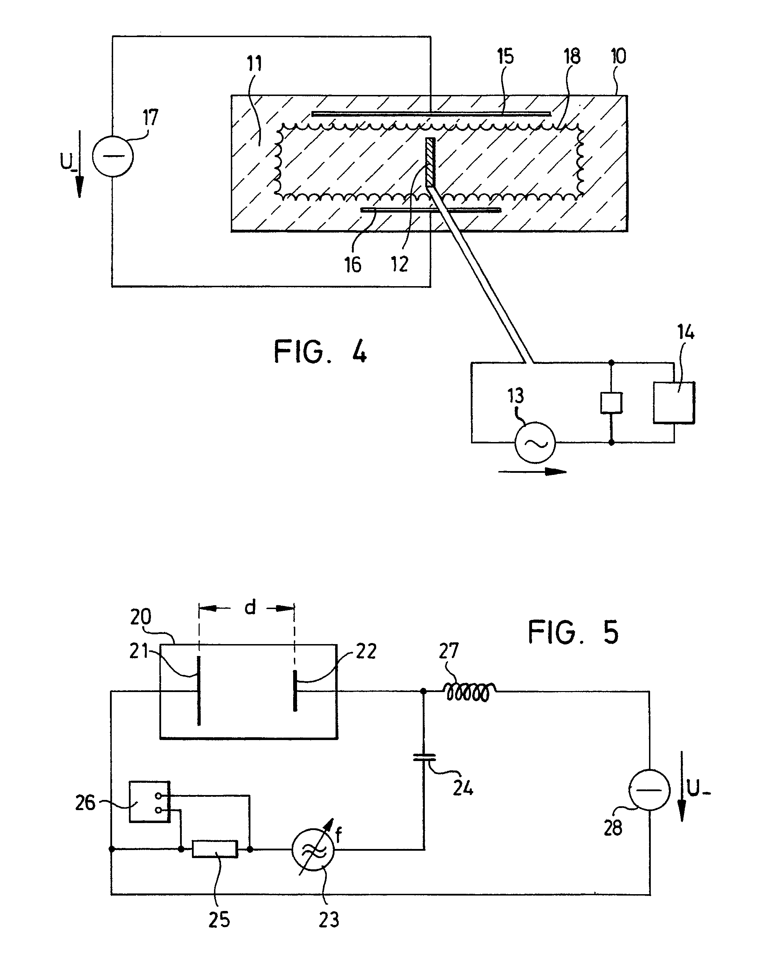

Die Messung von und die Beeinflussung mit Schwingungen innerhalb eines DNA- oder RNA-Moleküls kann bei Frequenzen von wenigen Hertz bis in die Größenordnung von 100 MHz und mehr grundsätzlich mechanisch (vibratorisch oder akustisch) erfolgen. Eine typische mechanische Koppelanordnung für das System DNA - Sonde Sender ist in Fig. 4 wiedergegeben. Bei dem dargestellten Ausführungsbeispiel ist eine Küvette 10 zur Aufnahme einer Probe 11, beispielsweise aus einem DNA-Material mit vorbekannter Basensequenz, vorgesehen. Ein vorzugsweise weitgehend resonanzfreier, breitbandiger akustischer Dünnschichtschwinger 12 taucht in die Probe 11 ein. Er ist an den Ausgang eines durchstimmbaren Wechselspannungsgenerators 13 angeschlossen. Der Generator 13 kann so ausgelegt sein, daß er Schwingungsgemische liefert. Stattdessen können auch mehrere Generatoren für Einzelfrequenzen zwecks Erzeugung von Schwingungsgemischen zusammengeschaltet sein. Im Ausgangskreis des Generators 13 liegt in Reihe mit dem Schwinger 12 eine Stromanzeigevorrichtung 14. In der Küvette 10 stehen sich zwei unterschiedlich große Kondensatorplatten 15 und 16 gegenüber. An den Kondensatorplatten liegt eine von einem Gleichspannungsgenerator 17 erzeugte Gleichspannung U__ an. Die Kondensatorplatten 15,16 erzeugen ein inhomogenes elektrisches Feld, in dem sich die DNA-Moleküle weitgehend parallel zueinander ausrichten. Bei 18 ist ein im Feld der Kondensatorplatten 15, 16 liegender, den Schwinger 12 umgreifender Schallabsorber angedeutet. Dessen Aufgabe ist es, störende Einflüsse der Küvette 10 auf das Schwingungsverhalten der den Schwinger 12 umgebenden Probe 11 zu beseitigen. Beim Durchstimmen des Wechselspannungsgenerators 13 wird das Probenmaterial 11 von dem Schwinger 12 zu erzwungenen Schwingungen veranlaßt. In Abhängigkeit von der von der Probe aufgenommenen Energie ändert sich der an der Stromanzeigeeinrichtung 14 angezeigte Erregerstrom des Schwingers 12. Dadurch läßt sich das Resonanzschwingungen auslösende Frequenzmuster (das angepaßte Signal) ermitteln.

Eine entsprechende Vorrichtung kann auch benutzt wurden, um auf DNA- oder RNA-Material an vorbestimmter Strangstelle einzuwirken. In diesem Fall wird der breitbandige Schwinger 12 zweckmäßig durch eine Gruppe von Schwingern ersetzt, die auf die Frequenz des für die betreffende Strangstelle spezifischen Schwingungsgemisches abgestimmt sind und die von einem das angepaßte Signal bereitstellenden Mustergenerator parallel oder einzeln angesteuert werden. Resonanzschwinger eignen sich insbesondere dazu, an der vorbestimmten Strangstelle einen gewollten Strangbruch herbeizuführen.

Ein typisches Beispiel für eine elektrische Koppelanordnung ist in Fig. 5 veranschaulicht. Ein Gefäß 20 aus isolierendem Werkstoff bildet zusammen mit Elektroden 21 und 22, von denen die eine 21 als Kondensatorplatte und die andere 22 als der Kondensatorplatte im Abstand d gegenüberstehende Spitze ausgebildet ist, einen Kondensator. Dieser wird aus einem Multischwingungs-Wechselspannungsgenerator 23 über einen mit dem Generator 23 in Reihe liegenden Koppelkondensator 24 gespeist. Im Wechselspannungs-Speisekreis liegt ferner ein Meßwiderstand 25, dem ein frequenzselektiver Spannungsmesser 26 parallelgeschaltet ist. Die Elektroden 21, 22 sind außerdem über eine HF-Drossel 27 an einen Gleichspannungsgenerator 28 angeschlossen. Zum Ermitteln des angepaßten Signals für eine bestimmte Basensequenz kann das Gefäß 20 beispielsweise mit hochreinen DNA-Molekülen aus einem einzigen Klon oder synthetisierter geringer Länge von z.B. nur 10 bis 20 Basenpaaren gefüllt werden. Entweder fährt man mit eingeprägter Spannung und mißt frequenzabhängig den Strom nach dem Modell eines Serienresonanzschwingers, oder umgekehrt nach dem Modell eines Parallelresonanzschwingers. Auch Kombinationen beider Schwingertypen kommen in Betracht. Die Anordnung gemäß Fig. 5 eignet sich insbesondere für den Fall, daß die angepaßten Signale im radarfrequenten Bereich liegen. Der Abstand d zwischen den Elektroden 21 und 22 sollte vorzugsweise kleiner als das 0,1-fache der Wellenlänge der elektrischen Schwingungen innerhalb der Küvette 20 sein. Dadurch werden mögliche Störungen durch Ausbreitungsphänomene vermieden. Die Elektroden bzw. Kondensatorplatten 21 und 22 unterscheiden sich vorzugsweise in ihren Abmessungen merklich, und zwar zweckmäßig um einen Faktor von mindestens 1:5, um ein inhomogenes elektrisches Feld zum Ausrichten der DNA- oder RNA-Moleküle in der lösung auszubilden.

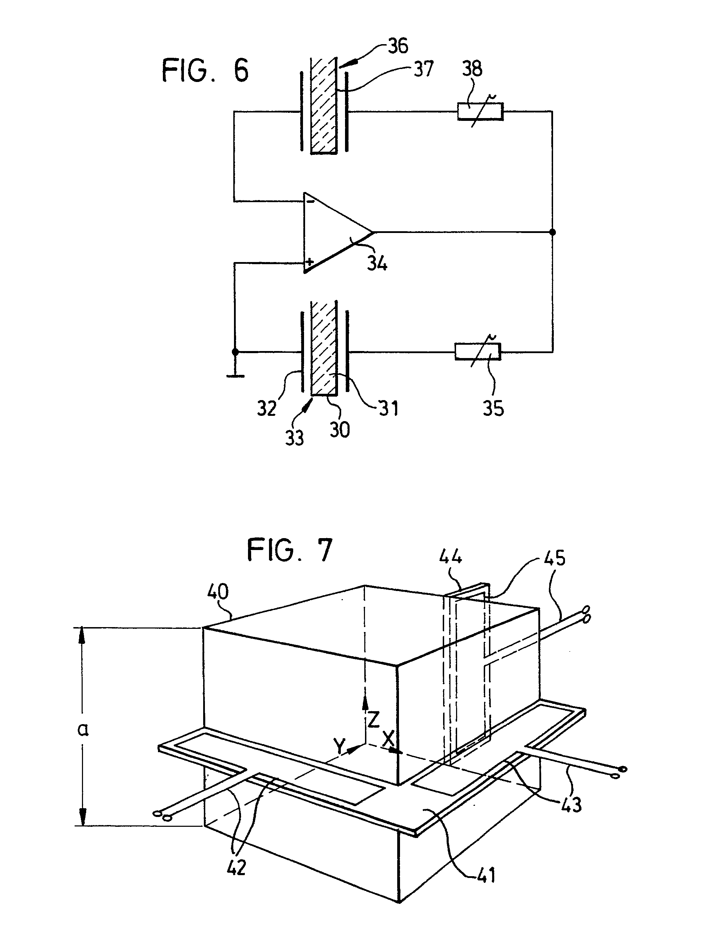

Eine weitere Möglichkeit besteht darin, eine Resonatoranordnung ähnlich Fig. 5 in einem rückgekoppelten Kreis eines Verstärkersystems nach der Methode des negativen Widerstandes zu betreiben, wie dies in Fig. 6 gezeigt ist. Dabei nimmt eine Küvette 30 z.B. ein Gemisch 31 von DNA-Material auf, von dem ein Teil eine Basensequenz hat, auf die eingewirkt werden soll, während bei einem anderen Teil des DNA-Materials keine solche Basensequenz vorliegt. Bei dem genannten ersten Teil kann es sich um DNA-Moleküle oder um Zellen mit diesen DNA-Molekülen handeln, die durch Herbeiführen eines Strangbruchs zerstört werden sollen. Der genannte andere Teil kann aus DNA-Material bestehen, das unbeeinflußt bleiben oder geschützt werden soll. Die Küvette 30 und ihr Inhalt 31 sitzen zwischen zwei einander gegenüberstehenden Kondensatorplatten 32. Der so erhaltene Resonator 33 liegt im Mitkopplungszweig eines Verstärkers 34. Diese Anordnung kann in dem DNA-Material 31 einen Schwingungszustand mit einem dem betreffenden Basenpaarmuster eigentümlichen Schwingungs- und Transientenmuster entstehen lassen. Durch Intensivieren der Verstärkung kann gegebenenfalls die gewünschte Resonanzkatastrophe in dem Gemisch 31 herbeigeführt werden. Damit nun aber nicht jede DNA des Gemisches 31 zerstört wird, ist dafür zu sorgen daß Resonanz nur für eine vorgegebene Basensequenz eintritt. Für diesen Zweck kann in Reihe mit dem Resonator 33 eine Dämpfungsfilteranordnung 35 gelegt sein, die bewirkt, daß sich in dem Mitkopplungszweig des Verstärkersystems 34 bevorzugt das an die vorgegebene Basensequenz angepaßte Signal aufbaut. Stattdessen oder zusätzlich kann das Verstärkersystem 34 in der in Fig. 6 veranschaulichten Weise als Differenzverstärker ausgelegt sein, in dessen Gegenkopplungszweig vorteilhaft ein dem Resonator 33 des Mitkopplungszweiges entsprechender Resonator 36 mit einer Küvette 37 liegt, welche die zu schützende DNA enthält. An Stelle des Resonators 36 oder, wie gezeigt, in Reihe mit diesem, kann eine variable Dämpfungsfilteranordnung 38 vorgesehen sein. Die Filteranordnung 38 kann für eine gezielte Dämpfung derjenigen Signalanteile ausgelegt sein, die im Resonator 31 eine Zerstörung der zu schützenden DNA herbeizuführen suchen.

Elektrische Koppelanordnungen nach Art der Vorrichtungen der Fign. 5 und 6 setzen relativ niederfrequente Eigenschwingungsgemische der DNA- oder RNA-Moleküle voraus, etwa bis in den Bereich von 100 bis höchstens 1000 GHz, was Vakuumwellenlängen im Bereich von 3 bis 0,3 mm entspricht.

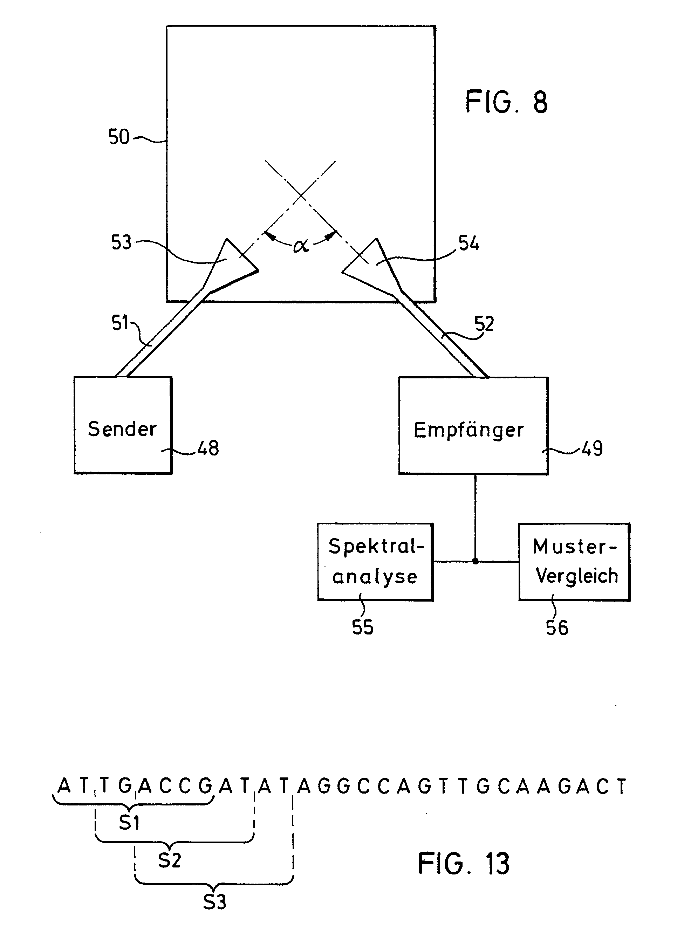

Im Bereich des Submillimeterwellen und erst recht im Bereich thermischer, sichtbarer oder noch kürzerer Wellenlängen (UV-Strahlung und weiche Röntgenstrahlung) kann mit Strahlungskopplungsvorrichtungen gearbeitet werden. Diese sollten für eine Kopplung zwischen dem Signal und dem DNA- oder RNA-Material sorgen, die möglichst frei von Gefäßeigenresonanzen ist, falls das betreffende Material in einem Gefäß untergebracht ist. Auf die Vermeidung von Gefäßresonanzen ist insbesondere bei elektrischen und magnetischen Koppelanordnungen (Fign. 7,8 und 9) zu achten, wenn die Abstände der Gefäßwände bei Vielfachen der Wellenlängen liegen. In dieser Beziehung unproblematischer sind Einrichtungen (Fign. 8, 9 und 10), die nach dem Reflexions-, Durchscheine- oder Maser-Prinzip arbeiten.

Fig. 7 zeigt eine magnetische Koppelanordnung zur Kopplung eines DNA- oder RNA-Volumens mit einer elektromagnetischen Sender/Empfänger-Einrichtung im Millimeter- und Submillimeterbereich. Eine Küvette 40 nimmt das Probenmaterial, z.B. DNA-Moleküle in wäßriger Lösung, auf. Die Abmessung a der Küvette 40 richtet sich nach dem zu untersuchenden Wellenlängenbereich. Ein an die Küvettenaußenwand angrenzender waagrechter Steg 41 trägt eine X-Rahmenantenne 42 und eine Y-Rahmenantenne 43. Auf einem lotrechten weiteren Steg 44 sitzt eine Z-Rahmenantenne 45. Die Antennen 42, 43 und 45 sind an eine nicht näher dargestellte Sender/Empfänger-Einrichtung angeschlossen. Die veranschaulichte Anordnung erlaubt es, Schwingungsspektren in allen drei Raumachsen X, Y und Z abzustrahlen bzw. aufzunehmen.

In Fig. 8 ist schematisch eine Meßvorrichtung zur Reflex-Strahlungskopplung dargestellt. Ein Sender 48 und ein Empfänger 49 sind an eine das Probenmaterial aufnehmende Küvette 50 über jeweils einen Wellenleiter 51 bzw. 52 und eine Strahlungsantenne 53 bzw. 54 angekoppelt, deren Achsen einen Winkel $\alpha$ miteinander bilden und sich innerhalb der Küvette 50 schneiden. Bei den Strahlungsantennen 53, 54 kann es sich beispielsweise um Hornstrahler handeln; die Wellenleiter 51, 52 können u.a. als Hohl- oder Streifenleiter ausgelegt sein. An den Ausgang des Empfängers 49 sind im veranschaulichten Ausführungsbeispiel eine Spektralanalyse-Einrichtung 55 und eine Mustervergleichs-Einrichtung 56 angeschlossen. Die Vorrichtung nach Fig. 8 erlaubt eine Ermittlung und elektrische Wirkungsmessung des oben diskutierten angepaßten Signals. Dabei werden in dem Empfänger 49 bzw. den daran angeschlossenen Einrichtungen 55, 56 um so höhere Reflexanteile gemessen, je besser das Sendesignal sequenzspezifisch angepaßt ist.

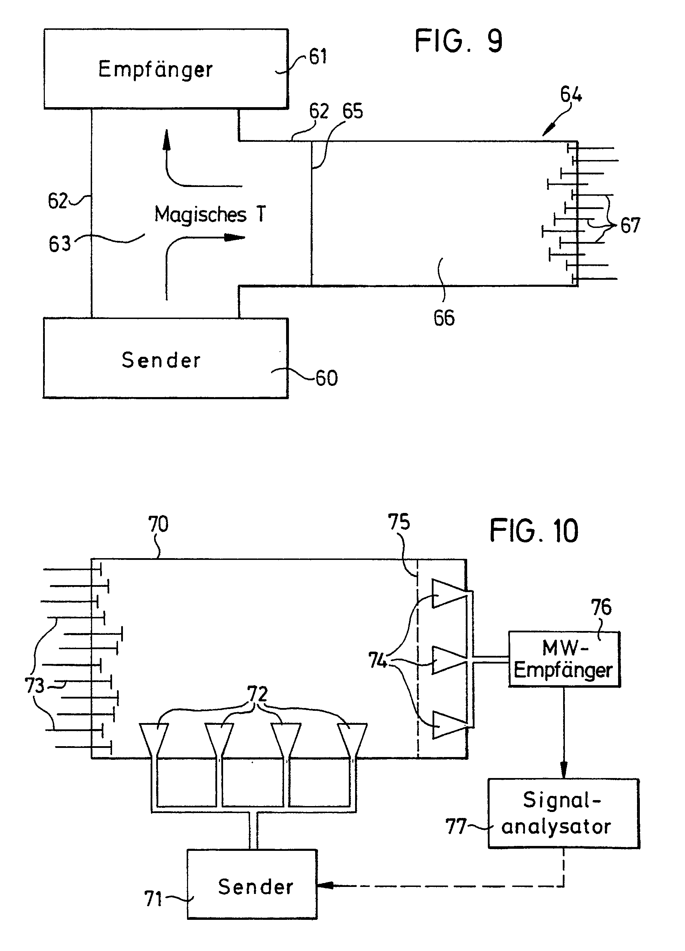

Fig. 9 zeigt eine Multiresonator-Koppelanordnung, bei der ein Sender 60 und ein Empfänger 61 über ein leitende Wandungen 62 aufweisendes sogenanntes "magisches T" 63 an eine Resonatoranordnung 64 angekoppelt sind. Das magische T erlaubt es, den Reflexionsfaktor in der aus der Hohlleitertechnik an sich bekannten Weise zu bestimmen. Bei 65 ist eine dünne, isolierende, HF-durchlässige Membran dargestellt, die den zur Aufnahme der Probe, z.B. DNA-Material in wäßriger Lösung, bestimmten Raum 66 der Resonatoranordnung abgrenzt. An der der Membran 65 gegenüberliegenden Seite des Raumes 66 befindet sich eine Mehrzahl von mechanisch einstellbaren Stempeln 67. Diese erlauben es, die Resonatoranordnung 64 auf ein multifrequentes Schwingungsspektrum abzustimmen.

In Fig. 10 ist eine Multiresonatoranordnung zum Erzeugen und Messen von sequenzangepaßten Signalen veranschaulicht, die in Anlehnung an bekannte Laser- bzw. Maser-Prinzipien arbeitet.

Eine Küvette 70 aus isolierendem Material bildet einen das Probematerial aufnehmenden Resonator. Ein Sender 71 koppelt ein geeignetes Pumpfrequenzgemisch über eine Gruppe von Sendeantennen 72 in den Resonator ein. In der Küvette 70 stehen sich eine Mehrzahl von mechanisch einstellbaren Stempeln 73 und eine Gruppe von Empfangsantennen 74 gegenüber. Vor letzterer liegt ein halbdurchlässiger Mikrowellenspiegel 75. Die Stempel 73 bilden totale Spiegel. An die Empfangsantennen 74 ist ein Mikrowellen-Empfänger 76 angeschlossen, dem ein Signalanalysator 77 nachgeschaltet ist. Letzterer kann gegebenenfalls seinerseits den Sender 71 anteuern. Wie bei Laser-/Maser-Anordnungen üblich, wird mit höherer als der spezifischen Frequenz gepumpt. Falls das geeignete Pumpfrequenzgemisch im Infraroten liegt, wird es über eine Optik zugeführt.

Sollte bei den erläuterten Anordnungen die räumliche Entkopplung zwischen Sender und Empfänger unzureichend sein, kann auch für eine zeitliche Entkopplung zwischen beiden gesorgt werden.

Basensequenzspezifische Molekülteilschwingungen können in DNA- und RNA-Molekülen auch durch eine Elektronenstrahlkopplung angeregt und synchronisiert werden, die sich an die bekannte Funktion der Wanderfeldröhre anlehnt, bei welcher ein Elektronenstrahl mit einer parallel zu dem Elektronenstrahl geführten elektromagnetischen Welle in Wechselwirkung tritt. Vorliegend beruht eine Elektronenstrahlkopplung auf dem Prinzip, einen Elektronenstrahl möglichst nahe an einer Ansammlung von DNA- oder RNA-Molekülen vorbeizuführen und dabei aufgrund von Basenschwingungen zeitlich schwankende Felder zu erzeugen, die ihrerseits mit dem Elektronenstrahl in Wechselwirkung treten. Aufgrund einer solchen Wechselwirkung kommt es in dem Elektronenstrahl zu einer Elektronenpaketbildung mit Phasenfokussierung, wobei gleichzeitig die Elektronenstrahl-Paketbildungs-Synchronisation von angesprochenen Basenpaarsequenzabschnitten in in einer Mehrzahl von DNA- bzw. RNA-Molekülen erfolgt.

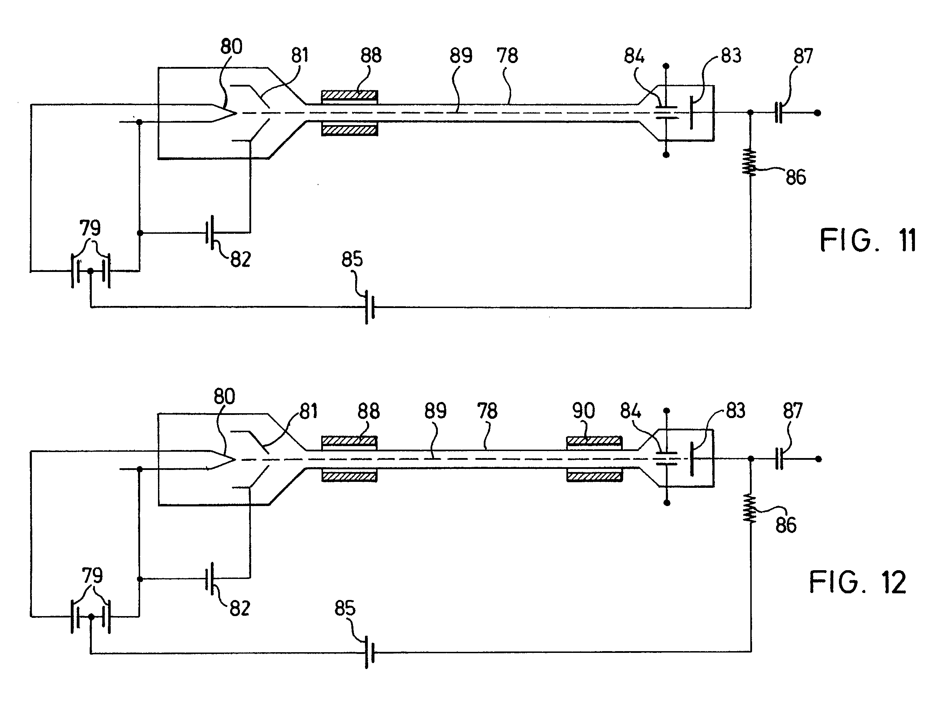

Für eine solche Elektronenstrahlkopplung eignet sich beispielsweise die in Fig. 11 veranschaulichte Vorrichtung. Letztere weist eine evakuierte Linearstrahlröhre 78 auf. Im Bereich des einen, in Fig. 11 linken Endes dieser Röhre ist eine Elektronenstrahlkanone untergebracht, zu der eine aus einer Heizspannungsquelle 79 versorgte, geheizte Kathode 80 und eine Steuerelektrode in Form eines Wehneltzylinders 81 gehören. Die Steuerelektrode ist gegenüber der Kathode 80 mittels einer Spannungsquelle 82 vorgespannt. Nahe dem anderen Ende der Röhre 78 befindet sich eine Anode 83, vor der ein Mikrokondensator 84 zur Schwingungsauskopplung sitzt. Die Anode 83 wird von einer Spannungsquelle 85 auf einem gegenüber der Kathode 80 positiven Potential gehalten. Mit der Spannungsquelle 85 ist zweckmäßig eine Drossel 86 in Reihe geschaltet. Eine verstärkte Ausgangswechselspannung kann über einen Koppelkondensator 87 abgenommen werden. Die Röhre 78 wird nahe der Elektronenstrahlkanone von einem ringförmigen Hohlraum 88 umfaßt. Dieser enthält diejenigen DNA- oder RNA-Moleküle, deren sequenzspezifisches Schwingungsmuster angeregt und gemessen werden soll.

Im Betrieb der Vorrichtung nach Fig. 11 erfährt der zunächst homogene Elektronenstrahl 89 unter dem Einfluß von einem ihm entgegenschwingenden Elektronenpaket, wie es die Basen der betreffenden RNA- oder DNA-Moleküle darstellen, abstoßende und damit verzögernde Kräfte. Dadurch erfolgt eine Paketbildung in dem Elektronenstrahl 89 selbst. Das so gebildete Elektronenpaket fliegt auf das Basen-Elektronenpaket etwas verlangsamt tangential zu und bringt letzteres vermöge verstärkter Abstoßwirkung zur Schwingungsumkehr. Daraufhin hört die Paketbildung in dem Elektronenstrahl momentan auf; es ensteht wieder ein nahezu homogener Elektronenstrahl. Der geschilderte Vorgang kann nun wieder von neuem beginnen und sich entsprechend periodisch wiederholen. In der Vorrichtung entsteht auf diese Weise ein Schwingungsgemisch, das für die Basensequenz des Probenmaterials in dem Hohlraum 88 eigentümlich ist. Das so gebildete Eigenschwingungsspektrum wird nach Amplitude und Phase gut meßbar.

Die in Fig. 12 veranschaulichte Vorrichtung unterscheidet sich von derjenigen nach Fig. 11 dadurch, daß zusätzlich zu dem kathodenseitigen Ringkanal 88 ein die Röhre 78 umgreifender weiterer Ringkanal 90 nahe dem anodenseitigen Röhrenende vorgesehen ist.

In den Ringkanal 88 können insbesondere kurze DNA- oder RNA-Moleküle mit einer vorbestimmten Basensequenz A eingebracht sein. Der Ringkanal 90 kann dagegen beispielsweise unterschiedliche und auch längere DNA- bzw. RNA-Moleküle enthalten, die gegebenenfalls in Zellen eingeschlossen sein können und von denen nur ein Teil die Basensequenz A aufweist. Ein für die Basensequenz A der Moleküle im Ringkanal 88 spezifisches Schwingungsmuster kann dann in der zuvor anhand der Fig. 11 erläuterten Weise erzeugt werden. Dieses Schwingungsmuster wird über den Elektronenstrahl 89 mit dem Molekülgemisch in dem Ringkanal 90 gekoppelt. Dabei werden diejenigen Moleküle des Gemisches, welche die Basensequenz A enthalten, im Bereich dieser Sequenz verstärkt zu Schwingungen angeregt. Es kann dadurch in diesen Molekülen gezielt ein Strangbruch am Ort der Basensequenz A herbeigeführt werden. Moleküle des Gemisches im Ringkanal 90 ohne die Basensequenz A bleiben dagegen unbeeinträchtigt.

Die vorstehend inbesondere anhand der Fign. 7 bis 12 erläuterten Koppelanordnungen erlauben es, mittels eines Signals, das an einen vorbestimmten Sequenzabschnitt angepaßt ist, gezielt mit diesem Sequenzabschnitt in Wechselwirkung zu treten. Dies läßt sich mit Vorteil auch für eine physikalische Sequenzanalyse durch stückweise fortschreitende Signalanpassung ausnutzen. Um dies zu erläutern, sei angenommen, daß ein DNA-Strang die in Fig. 13 dargestellte Sequenz enthält, von der zunächst nur der Sequenzabschnitt S1, d.h.

ATTGACCG

bekannt ist. Ist ferner für diesen Sequenzabschnitt S1 das angepaßte Signal bekannt, das im Zeitbereich mit $s_1(t)$ oder im Frequenzbereich als Spektrum $s_1(f)$ bezeichnet sei und wird dieses angepaßte Signal auf eine Probe aus gleichartigen DNA-Molekülen appliziert, die beispielsweise die vorstehend genannte Basensequenz haben, wird dem Sequenzabschnitt S1 relativ viel Energie zugeführt. Dabei bleibt man aber absichtlich unterhalb derjenigen Energie, die zum Strangbruch führt. Wegen der starken Energieaufnahme im Gebiet des Sequenzabschnittes S1 werden aufgrund von Nachbarschaftskoppeleffekten durch die realiter vorhandenen Nichtlinearitäten auch die Nachbarn rechts des Sequenzabschnitts S1 angeregt, d.h., es erscheinen nun zusätzliche Signale, die ursprünglich nicht angeregt waren.

Grundsätzlich kann dem Sequenzabschnitt S1 auf der in Fig. 13 rechten Seite einer von insgesamt sechzehn möglichen Zweierabschnitten, nämlich AA, AG, AT, AC, GA, GG, GT, GC, TA, TG, TT, TC, CA, CG, CT und CC, folgen. Kennt man auch die angepaßten Signale für die sechzehn denkbaren Kombinationen S1 + AA, S1 + AG usw. bis S1 + CC, kann durch einen Referenz-Mustervergleich herausgefunden werden, daß im vorliegenden Beispiel dem Sequenzabschnitt S1 genau der Zweierabschnitt AT folgt. Dann wird ein angepaßtes Signal $s_2(t)$ erzeugt, das dem um zwei Basen nach rechts gerückten Sequenzabschnitt S2 entspricht, also hier TGACCGAT. Nun wird das gefundene angepaßte Signal $s_2$ appliziert, wobei in der Meßvorrichtung nach den Fign. 7 bis 12 wieder neue nicht applizierte Signale erscheinen, und zwar dieses Mal gemäß den linken und den rechten Nachbarn des Sequenzabschnittes S2. Da die linken Nachbarn des Sequenzabschnittes S2 bekannt sind, kann man die neu hinzukommenden in Fig. 13 rechten Nachbarn in der gerade erläuterten Weise identifizieren und dann das auf den Sequenzabschnitt S3, nämlich ACCGATAT, angepaßte Signal erneut applizieren. Durch sinngemäße Fortsetzung dieses Verfahrens kann schließlich eine beliebig lange DNA-Kette sequenziert werden. Da die Suchstrategien algorithmisierbar sind, kann das Verfahren automatisiert werden. Somit können selbst größere Genome in viel kürzerer Zeit und wesentlich einfacher als es mittels der chemischen Sequenzanalyse möglich ist, sequenziert werden.

Bei den geschilderten Vorgehensweisen ist zu berücksichtigen, daß das angepaßte Signal zeitvariabel sein kann, wenn von außen Energie in Form von Wärme oder elektrischem, magnetischem oder elektromagnetischem Rauschen zugeführt wird. Man hat es dann mit einem Korrespondenzproblem zeitvariabler Muster zu tun. Korrespondenzen zwischen derartigen Mustern lassen sich durch die in der Nachrichtentechnik als das Konzept des "angepaßten Filters" (Faltung), der "Kreuzkorrelation" oder der "Maximum-Likelihood-Methode" bekannten Verfahren auffinden. Zweckmäßig sollte man mit den kürzesten (stabilen) DNA- oder RNA-Stücken als Versuchsmaterial beginnen, bzw. bei längeren Sequenzen mit weitgehend regelmäßigem Aufbau, wie z.B. AAATTTGGGCCC.

Um stärkere Meßsignale aus einer Meßanordnung für DNA- oder RNA-Moleküle zu gewinnen, kann es zweckmäßig sein, - wie Watson und Crick für die Röntgenstrukturanalyse - die (gleichartigen) DNA-Moleküle in lyophilisiertem Zustand in den Resonator einzubringen. Dabei ist zu berücksichtigen, daß sich je nach Wasserentzug der Grad der Regelmäßigkeit der Helixstruktur in Richtung auf die kristalline Form ändern kann.

Gegebenenfalls kann mit zunehmendem Wasserentzug auch ein Übergang der DNA- oder RNA-Moleküle von der biologisch aktiven A-Form in die B-Form erfolgen, wobei sich das angepaßte Signal für die A-Form von demjenigen für die B-Form unterscheiden kann.

Andererseits gibt es viele Anwendungsfälle, bei denen man das DNA- oder RNA-Material unter natürlichen Bedingungen behandeln und auch ohne Küvette oder einen vergleichbaren Behälter arbeiten wird. Die zusätzlich zur skizzierten Sequenzanalyse gegebenen praktischen Einsatzmöglichkeiten des vorstehend diskutierten Verfahrens sind vielfältig, wobei man sich zu vergegenwärtigen hat, daß bereits ein einzelner Strangbruch zur biologischen Inaktivität des betreffenden RNA- oder DNA-Moleküls bzw. der ein solches Molekül aufweisenden Zelle führt. Durch Applikation des angepaßten Signals können daher gezielt diejenigen Zellen unwirksam gemacht werden, die den zu dem angepaßten Signal gehörenden Sequenzabschnitt aufweisen und die sich durch diesen Sequenzabschnitt von anderen Zellen unterscheiden, welche nicht beeinflußt werden sollen. Beispielsweise lassen sich auf diese Weise abartige oder virusinfizierte Zellen abtöten. Mit den beschriebenen Einrichtungen als Grundbausteinen läßt sich u.a. eine Blutwaschanordnung aufbauen, die Zellen mit fremdem Erbgut ausschaltet. Ein weiteres Anwendungsgebiet ist u.a. die selektive Pflanzenschädlingsbekämpfung mit Hilfe eine angepaßte Signale abstrahlenden Anordnung, mit der an den zu schützenden Pflanzen im Nahbereich vorbeigefahren wird. Dabei sind die angepaßten Signale auf spezielle Sequenzabschnitte der DNA von Schädlingen abzustimmen, die in den zu schützenden Pflanzen nicht vorkommen.

Charakteristische DNA- und RNA-Spektrallinien sind insbesondere im längerwelligen Infrarotbereich und im Submillimeterbereich zu erwarten. Eine Wechselwirkung zwischen einer Strahlenquelle oder einem Strahlendetektor und dem von dem DNA- oder RNA-Material gebildeten Schwingungssystem ist aber wegen der hohen Dämpfungswerte, die Wasser in diesem Frequenzbereich hat, sehr ershwert, wenn nicht gar praktisch unmöglich, falls die DNA- oder RNA-Moleküle von Wasser umgeben sind. Die Verwendung wasserfreier DNA- oder RNA-Substanzen scheidet für die wichtigen Anwendungen im Bereich biologisch aktiver DNA oder RNA aus, weil Wasser für die DNA oder RNA eine natürliche Umgebung darstellt und insbesondere in lebenden Zellen DNA und RNA immer in wäßriger Lösung vorliegt.

Dieser Schwierigkeit kann vorliegend dadurch begegnet werden, daß das Probematerial mit Schwingungen in einem für Wasser dämpfungsarmen Wellenlängenbereich, etwa im kürzerwelligen Infrarotbereich oder im sichtbaren Bereich, beaufschlagt und die Nichtlinearität des komplexen DNA- oder RNA-Schwingers genutzt wird, um durch parametrische Interaktion selbsttätig Summenfrequenzen, Differenzfrequenzen und/oder höhere Harmonische zu bilden, welche ungeachtet des Umgebungsmediums Wasser das Probematerial zu Eigen- oder Resonanzschwingungen anregen und/oder das Erfassen der Eigenschwingungsfrequenz des Probematerials gestatten. Das Prinzip der parametrischen Schwingungserzeugung ist aus der Ramanspektroskopie, aus der Modulationstechnik der Nachrichtentechnik und aus der energiereichen hydro-akustischen Niederfrequenzschallerzeugung von einer langen linienhaften Schallabstrahlbasis an sich bekannt. Es beruht auf der Tatsache, daß zwei Schwingungen $f_1$ und $f_2$, die auf ein nichtlineares Element treffen, Kombinationsfrequenzen der allgemeinen Form

$$nf_1 \pm mf_2$$

erzeugen, wobei n und m ganze positive Zahlen sind. Zum Beispiel kann die Interaktion mit einem komplexen DNA-Schwingungssystem, das durch Eigenfrequenzen im Submillimeterbereich, beispielsweise die Frequenzen $f_{S1}$, $f_{S2}$ ... $f_{Sn}$ gekennzeichnet ist, wie folgt durchgeführt werden:

Strahlungsquellen strahlen in einem für Wasser dämpfungsarmen Wellenlängenbereich, etwa im kurzwelligen infraroten oder im sichtbaren Bereich, mit Frequenzen $f_H$, $f_{H1}$, $f_{H2}$ ... $f_{Hn}$ auf eine DNA-Probe oder auf DNA enthaltende Zellen, wobei

$$f_{H1} = f_H - f_{S1}$$
$$f_{H2} = f_H - f_{S2}$$
.
.
$$f_{Hn} = f_H - f_{Sn}$$

ist. Am nichtlinearen Schwingungssystem der DNA entstehen dann die gewünschten

Frequenzen $f_{S1}$, $f_{S2}$ ... $f_{Sn}$ als Differenzfrequenzen

$$f_H - f_{H1} = f_{S1}$$
$$f_H - f_{H2} = f_{S2}$$
.
.
$$f_H - f_{Hn} = f_{Sn}.$$

Die Frequenz $f_H$ ist dabei zur Vermeidung von im Submillimeterbereich liegenden unerwünschten Kombinationsfrequenzen so zu wählen, daß keine der Frequenzen $f_{H1}$ bis $f_{Hn}$ in den von $f_{S1}$ bis $f_{Sn}$ reichenden Frequenzbereich fällt, also die Bedingung

$$f_H > f_{H1} + f_{Sn}$$

erfüllt ist.

Während vorstehend Frequenzen $f_{H1}$ bis $f_{Hn}$ benutzt werden, die Kleiner als $f_H$ sind, kann auch mit Frequenzen $f_{H1}$ bis $f_{Hn} > f_H$ gearbeitet werden, wobei dann gilt:

$$f_{H1} = f_H + f_{S1}$$
$$f_{H2} = f_H + f_{S2}$$
.
.
$$f_{Hn} = f_H - f_{Sn}$$

sowie entsprechend:
$$f_{H1} - f_H = f_{S1}$$
$$f_{H2} - f_H = f_{S2}$$
.
.
$$f_{Hn} - f_H = f_{Sn}.$$

Zum Erkennen und Messen der Amplituden der DNA- oder RNA-Eigenschwingungen kann auch lediglich eine einzelne Schwingung der Frequenz $f_H$

auf die Probe gestrahlt werden. Aufgrund der Nichtlinearität des DNA- oder RNA-Schwingers entstehen dann Frequenzen

$$f_H + f_{S1}, f_H + f_{S2}, .... f_H + f_{Sn}$$

bzw.

$$f_H - f_{S1}, f_H - f_{S2} .... f_H - f_{Sn},$$

die bei geeigneter Wahl von $f_H$ in einen für Wasser dämpfungsarmen Wellenlängenbereich fallen und daher leicht detektiert werden können.

Grundsätzlich kann eine solche parametrische Interaktion auch durch Beaufschlagen mit einer oder mehreren Frequenzen $f_H$ bzw. $f_H$, $f_{H1}$, $f_{H2}$ ... $f_{Hn}$ erfolgen, die niedriger als die Frequenzen $f_{S1}$ bis $f_{Sn}$ sind, wobei dann wegen der Nichtlinearität des Schwingers entstehende höhere Harmonische genutzt werden.

**Patentansprüche**

1. Verfahren zum Ermitteln eines für eine bestimmte Basenpaarsequenz von DNA- oder RNA-Molekülen spezifischen Schwingungsgemisches nach Amplitude und Phase, bei dem das DNA- bzw. RNA-Material, das die bestimmte Basenpaarsequenz hat, Schwingungen unterschiedlicher Frequenz ausgesetzt oder zu Eigenschwingungen angeregt wird und die Frequenz oder Frequenzen nach Amplitude und Phase ermittelt werden, bei denen in dem DNA- bzw. RNA-Material Resonanzschwingungen auftreten.

2. Verfahren zum Untersuchen von DNA- oder RNA-Molekülen auf das Vorhandensein einer bestimmten Basenpaarsequenz, bei dem die Moleküle durch erzwungene Schwingungen oder Eigenschwingungen mit einem nach Amplitude und Phase vorbestimmten Schwingungsgemisch angeregt werden, das spezifisch für die gesuchte Basenpaarsequenz ist, und bei dem ermittelt wird, ob beim Anregen mit diesem Schwingungsgemisch Resonanzschwingungen auftreten.

3. Verfahren zum Einwirken auf DNA- oder RNA-Moleküle an vorgewählter Strangstelle durch Beaufschlagen der Moleküle mit einem nach Amplitude und Phase vorbestimmten, basenpaarsequenzspezifischen Gemisch von erzwungenen Schwingungen oder von Eigenschwingungen.

4. Verfahren nach Anspruch 3, dadurch gekennzeichnet, daß den DNA- oder RNA-Molekülen mittels des basenpaarsequenz-spezifischen Schwingungsgemisches eine Energiemenge zugeführt wird, die einen Strangbruch an der vorgewählten Strangstelle verursacht.

5.  Verfahren nach Anspruch 3, dadurch gekennzeichent, daß die DNA- oder RNA-Moleküle an einem ersten Sequenzabschnitt, der eine bekannte Sequenz von n Basenpaaren hat, durch ein für diese Basenpaarsequenz spezifisches Schwingungsgemisch mit einer nicht zum Strangbruch führenden Energie zu Resonanzschwingungen angeregt werden, das Spektrum der in den DNA- bzw. RNA-Molekülen angeregten Resonanzschwingungen nach Amplitude und Phase ermittelt und mit bekannten Resonanzschwingungsspektren verglichen wird, die spezifisch für eine Sequenz von n + m Basenpaaren sind, wobei n und m ganze Zahlen sind, und anhand dieses Vergleichs die Basenpaarsequenz für einen zweiten Sequenzabschnitt ermittelt wird, der gegenüber dem ersten Sequenzabschnitt entlang dem Strang um m Basenpaare versetzt ist; die DNA-bzw. RNA-Moleküle dann mit einem nicht zum Strangbruch führenden Schwingungsgemisch angeregt werden, das spezifisch für die Basenpaarsequenz des zweiten Sequenzabschnittes ist; und die vorgenannten Verfahrensschritte bedarfsweise wiederholt werden.

6.  Verfahren nach Anspruch 5, dadurch gekennzeichnet, daß m Kleiner als n gewählt wird.

7.  Verfahren nach Anspruch 5 oder 6, dadurch gekennzeichnet, daß der zweite Sequenzabschnitt so gewählt wird, daß er die gleiche Anzahl von n Basenpaaren wie der erste Sequenzabschnitt hat.

8.  Verfahren nach einem der vorhergehenden Ansprüche, dadurch gekennzeichnet, daß das DNA- oder RNA-Material während der Behandlung oder Untersuchung abgekühlt oder in lyophilisiertem Zustand gehalten wird.

9.  Verfahren nach einem der vorhergehenden Ansprüche, dadurch gekennzeichnet, daß das DNA- oder RNA-Material in wäßriger Lösung behandelt und/oder untersucht wird.

10. Verfahren nach einem der vorhergehenden Ansprüche, dadurch gekennzeichnet, daß die DNA- oder RNA-Moleküle mittels eines inhomogenen elektrischen Gleichfeldes weitgehend parallel zueinander ausgerichtet werden.

11. Verfahren nach Ansprüchen 8 und 10, dadurch gekennzeichnet, daß die Ausrichtung während des Lyophilisierens erfolgt.

12. Verfahren nach einem der vorhergehenden Ansprüche, dadurch gekennzeichnet, daß die Beaufschlagung des DNA- oder RNA-Materials mit Schwingungen und/oder die Messung des basenpaarsequenz-spezifischen Resonanzschwingungsspektrumsmechanisch, insbesondere vibratorisch oder akustisch, erfolgen.

13. Verfahren nach einem der vorhergehenden Ansprüche, dadurch gekennzeichnet, daß das Anregen des DNA- oder RNA-Materials zu erzwungenen Schwingungen oder zu Eigenschwingungen und/oder das Ermitteln des basenpaarsequenz-spezifischen Resonanzschwingungsspektrums durch kapazitives Ankoppeln eines Schwingungssenders bzw. einer Entdämpfungsvorrichtung oder eines Schwingungsempfängers erfolgen.

14. Verfahren nach einem der Ansprüche 1 bis 12, dadurch gekennzeichnet, daß das Anregen des DNA- oder RNA-Materials zu erzwungenen Schwingungen oder zu Eigenschwingungen und/oder das Ermitteln des basenpaarsequenz-spezifischen Resonanzschwingungsspektrums durch magnetisches oder elektromagnetisches Ankoppeln eines Schwingungssenders bzw. einer Entdämpfungsvorrichtung oder eines Schwingungsempfängers erfolgen.

15. Verfahren nach einem der Ansprüche 1 bis 12, dadurch gekennzeichnet, daß das Anregen des DNA- oder RNA-Materials zu erzwungenen Schwingungen oder zu Eigenschwingungen und/oder das Ermitteln des basenpaarsequenz-spezifischen Resonanzschwingungsspektrums durch Strahlungsankopplung eines Schwingungssenders bzw. einer Entdämpfungsvorrichtung oder eines Schwingungsempfängers erfolgen.

16. Verfahren nach einem der vorhergehenden Ansprüche, dadurch gekennzeichnet, daß zum Anregen des DNA- oder RNA-Materials zu erzwungenen Schwingungen oder zu Eigenschwingungen und/oder zum Ermitteln des basenpaarsequenz-spezifischen Resonanzschwingrungsspektrums das DNA- oder RNA-Material mit einer oder mehreren Schwingungen, deren Frequenz in einem für Wasser dämpfungsarmen, von dem Bereich der Eigen- oder Resonanzschwingungen des DNA- oder RNA-Materials in Abstand befindlichen Frequenzbereich liegt, zu parametrischer Interaktion gebracht wird.

17. Vorrichtung zur Durchführung des Verfahrens nach Anspruch 1, 2 oder 16, gekennzeichnet durch eine DNA- oder RNA-Moleküle zu erzwungenen Schwingungen oder Eigenschwingungen mit variabler Frequenz veranlassende Anregungseinrichtung sowie durch eine Meßeinrichtung zum Ermitteln des Schwingungsgemisches nach Amplitude und Phase, bei dem die DNA- bzw. RNA-Moleküle in Resonanzschwingungen kommen.

18. Vorrichtung zur Durchführung des Verfahrens nach einem der Ansprüche 3 bis 16, gekennzeichnet durch eine Anregungseinrichtung, die DNA- oder RNA-Moleküle zu Resonanzschwingungen veranlaßt.

19. Vorrichtung nach Anspruch 17 oder 18, dadurch gekennzeichnet, daß die Anregungseinrichtung einen Mustergenerator zur Herbeiführung von erzwungenen Schwingungen der DNA- oder RNA-Moleküle mit einem nach Amplitude und Phase vorbestimmten oder einstellbaren Schwingungsspektrum aufweist.

20. Vorrichtung nach einem der Ansprüche 17 bis 19, dadurch gekennzeichnet, daß die Anregungseinrichtung als Anordnung zum Entdämpfen des von den DNA- oder RNA-Molekülen gebildeten schwingungsfähigen Systems ausgelegt ist.

21. Vorrichtung nach Anspruch 20, dadurch gekennzeichnet, daß die Anregungseinrichtung einen rückgekoppelten Verstärker mit einem Mitkopplungszweig aufweist, in dem ein DNA- oder RNA-Material enthaltender Resonator liegt.

22. Vorrichtung nach Anspruch 21, dadurch gekennzeichnet, daß der rückgekoppelte Verstärker zusätzlich einen Gegenkopplungszweig aufweist, in dem ein Resonator mit DNA- oder RNA-Material liegt, das von dem DNA- oder RNA-Material in dem Resonator des Mitkopplungszweiges verschieden ist.

23. Vorrichtung nach einem der Ansprüche 17 bis 22, dadurch gekennzeichnet, daß die Anregungseinrichtung als elektroakustische Einrichtung mit einer Schwingeranordnung ausgebildet ist, die mit DNA- oder RNA-Material mechanisch koppelbar ist.

24. Vorrichtung nach einem der Ansprüche 17 bis 22, dadurch gekennzeichnet, daß die Anregungseinrichtung als an das DNA- oder RNA-Material kapazitiv, magnetisch oder elektromagnetisch ankoppelbare Schwingungseinrichtung ausgebildet ist.

25. Vorrichtung nach einem der Ansprüche 17 bis 22, dadurch gekennzeichnet, daß die Anregungseinrichtung als an das DNA- oder RNA-Material durch Strahlungskopplung ankoppelbare Schwingungseinrichtung ausgebildet ist.

26. Vorrichtung nach Anspruch 25, gekennzeichnet durch eine nach dem Reflexions-, Durchscheine- oder Maser-Prinzip arbeitende Strahlungskopplung.

27. Vorrichtung nach Anspruch 25, gekennzeichnet durch eine Elektronenstrahlkopplung.

28. Vorrichtung nach einem der Ansprüche 17 bis 27, gekennzeichnet durch Mittel zum gegenseitigen Ausrichten der zu Schwingungen anzuregenden DNA- oder RNA-Moleküle, insbesondere in Form einer Einrichtung zum Erzeugen eines inhomogenen elektrischen Gleichfeldes.

FIG. 1

FIG. 2

# FIG. 3

FIG. 4

FIG. 5

FIG. 6

FIG. 7

FIG. 8

A T T G A C C G A T A T A G G C C A G T T G C A A G A C T

S1

S2

S3

FIG. 13

FIG. 9

Empfänger — 61

62

63

Magisches T

Sender — 60

62 65

64

67

66

FIG. 10

70

75

73

72

74

76

MW-Empfänger

77

Signal-analysator

71

Sender

FIG. 11

FIG. 12